**Europäisches Patentamt**

**European Patent Office**

**Office européen des brevets**

(19)

(11) Veröffentlichungsnummer: **0 125 322**
**B1**

(12) **EUROPÄISCHE PATENTSCHRIFT**

(45) Veröffentlichungstag der Patentschrift:
24.09.86

(21) Anmeldenummer: **83104643.8**

(22) Anmeldetag: **11.05.83**

(51) Int. Cl.⁴: **A 23 K 1/16**

(54) Verfahren zur Anwendung ergotroper Wirkstoff-Kombinationen in der Aufzucht und Mast von Nutztieren.

(43) Veröffentlichungstag der Anmeldung:
21.11.84 Patentblatt 84/47

(45) Bekanntmachung des Hinweises auf die Patenterteilung:
24.09.86 Patentblatt 86/39

(84) Benannte Vertragsstaaten:
AT CH DE FR LI SE

(56) Entgegenhaltungen:
DD-A-126 626
DE-A-2 437 155

(73) Patentinhaber: VEB Berlin- Chemie, Glienicker Weg 125- 127, DDR- 1199 Berlin (DD)

(72) Erfinder: Just, Helmut, Dr., Vetschauer Allee 4, DDR- 1187 Berlin (DD)
Erfinder: Schinkowski, Klaus, Dr., Hörnlestrasse 40, DDR- 1170 Berlin (DD)
Erfinder: Tschauschev, Peter, Dr., Andreasstrasse 47, DDR- 1017 Berlin (DD)
Erfinder: Hackl, Wolfgang, Dr., Seestrasse 13, DDR- 2530 Rostock (DD)
Erfinder: Jackisch, Brigitte, Dr., Amalienstrasse 3, DDR- 3720 Blankenburg (DD)
Erfinder: Jeroch, Heinz, Dr., Karl- Marx- Strasse 34, DDR- 7152 Böhlitz- Ehrenberg (DD)
Erfinder: Michelchen, Gertrud, Dr., Budapester Strasse 17, DDR- 2500 Rostock (DD)
Erfinder: Poppe, Siegfried, Prof. Dr. sc.agr., Wallensteinstrasse 24, DDR- 2500 Rostock (DD)
Erfinder: Weber, Klaus, Dr., Nordstrasse 11, DDR- 3720 Blankenburg (DD)

(74) Vertreter: Hansen, Bernd, Dr.rer.nat., Hoffmann, Eitle & Partner Patentanwälte Arabellastrasse 4, D-8000 München 81 (DE)

## Beschreibung

Die Erfindung betrifft ein Verfahren zur Anwendung ergotroper Wirkstoffkombinationen in der Aufzucht und Mast von Nutztieren, z.B. von Geflügel, Schweinen und Rindern.

Die Erfindung betrifft insbesondere ein Verfahren zur Anwendung von Kombinationen von N-Guanidino-N'-thioureido-p-benzochinon-diimid sowie 6-Methyluracil in verschiedenen Mengerelationen.

Es ist bekannt, daß in der Aufzucht von Mast verschiedener Tierarten, wie Geflügel, Rinder und Schweine, Zusatzstoffe verwendet werden, deren Applikation an die Tiere als Bestandteil des Futters eine Reihe von Vorteilen bringt. So kann damit das Wachstum der Tiere beschleunigt werden und sie erreichen früher das erforderliche Schlachtgewicht. Weiterhin kann damit eine bessere Ausnutzung des Futters erreicht werden; dieser Effekt führt im Ergebnis der Mast zu einem geringeren Futteraufwand.

Die Natur solcher ergotrop wirkender Substanzen umfaßt ein weites Spektrum verschiedenartiger chemischer Strukturen. An erster Stelle sind die Antibiotika, wie Penicilline, Tetracycline, Oxytetracyclin, Zinkbacitracin, Oleandomycin, Flavomycin, Salinomycin, Tylosin u. a. zu nennen. Darüber hinaus werden weitere antibakteriell wirksame Stoffe nicht natürlichen Ursprungs wie Chinoxalin-N,N'-dioxide, z. B. Carbadox, Quindoxin und Olaquindox sowie Stoffe vom Typ des Nitrofurans wie beispielsweise Furazolidon und Nitrovin eingesetzt. Darüber hinaus können eine ganze Reihe weiterer Stoffe natürlichen und synthetischen Ursprungs, wie beispielsweise bestimmte Metallverbindungen von Kupfer, Mangen, Zink oder 3-Nitro-4-hydroxyphenylarsonsäure oder Östrogene wie Diäthylstilböstrol zur Erzielung ergotroper Wirkungen eingesetzt werden.

Weiterhin ist durch die DE-OS 2.437155 sowie durch RAAKE u.a. (Berliner und Münchener Tierärztliche Wochenschrift 88,188 (1975) die Möglichkeit zur Verwendung von 6-Methyluracil (Pseudothymin) als Ergotropikum bei verschiedenen Tierarten bekannt geworden. Die zur Erzielung eines optimalen Zuwachses erforderlichen Mengen an Pseudothymin betragen dabei 100 - 200 ppm im Futter.

Weiterhin ist in der DD 126.626 vorgeschlagen worden, daß bestimmte Derivate das p-Benzochinons, vornehmlich substituierte p-Benzochinondiimine, die an beiden N-Atomen weitere Substituenten tragen, wobei bevorzugte Verbindungen N-Guanidino-N'- (4-methylsemicarbazono)-p-benzochinondiimid, N-Guanidino-N'-thioureido-p-benzochinondiimid u.a. sind, in Mengen von 1-50 ppm im Futter bei Broiler und Schweinen einen das Wachstum stimulierenden Effekt ausüben können.

Ein Nachteil vornehmlich der Antibiotika und antimikrobiell wirkenden Ergotropika ist die Tatsache, daß sich nach einer gewissen Anwendungszeit der durch diese Stoffe erzielbare Wachstumseffekt beträchtlich verringert. Dadurch wird der Einsatz dieser Stoffe hinsichtlich ihres ergotropen Effekts uneffektiv und es müssen andere Ergotropika eingesetzt werden.

Diese Umstellungen erfordern in der Produktion der Wirkstoffe erhebliche Anstrengungen technischer und finanzieller Art für neue Wirkstoffe, da der volkswirtschaftliche Bedarf sehr hoch ist.

Ein weiterer Nachteil der als Ergotropika eingesetzten Wirkstoffe mit antimikrobieller Wirkung besteht in der Herausbildung von Kreuzresistenzen der Bakterien in der Darmflora gegenüber therapeutisch angewandten Stoffen wie Sulfonamiden und deren Kombinationen mit Trimethoprim bzw. Diaveridin, Chloramphenicol, Penicillinen und anderen Antibiotika. Diese durch den langandauernden Einsatz antimikrobiell wirkender Ergotropika entstehende Kreuzresistenz verhindert den im Krankheitsfall erforderlichen therapeutischen Effekt bei Einsatz anderer Antibiotika und ähnlich wirkender Stoffe.

Das Ziel der Erfindung liegt darin, ein Verfahren zur Anwendung neuer Ergotropika aufzuzeigen, bei dem die angeführten Nachteile beseitigt sind.

Der Erfindung liegt die Aufgabe zugrunde, neue Kombinationen von Wirkstoffen in der Aufzucht und Mast von Nutztieren, z.B. von Geflügel, Schweinen und Rindern anzuwenden, bei denen die Einsatzmengen gegenüber der Einzelanwendung beträchtlich verringert werden können.

Es wurde nun erfindungsgemäß festgestellt, daß durch Kombination bestimmter Wirkstoffe deren ergotroper Effekt (Mastendgewicht) gesteigert werden kann, wobei erfindungsgemäß die Konzentration jedes Wirkstoffes gegenüber den jeweiligen Konzentrationen bei Einzelapplikation erniedrigt ist. Es wurde weiterhin erfindungsgemäß festgestellt, daß der ergotrope Effekt der Kombinationen, die die Wirkstoffe erfindungsgemäß in geringer Konzentration als bei der Einzelanwendung beschrieben, enthalten, größer ist als bei der Einzelapplikation derselben Wirkstoffe. Dies ist überraschend, da beispielsweise in der DD 126.626 gezeigt wird (Beispiel 5), daß mit sinkenden Konzentrationen des Wirkstoffes (N-Guanidino-N'-thioureido-p-benzochinon-diimid) auch der Masteffekt deutlich geringer wird.

Es wurde weiterhin erfindungsgemäß festgestellt, daß dieser Effekt bevorzugt bei der Kombination von 6-Methyluracil mit N-Guanidino-N'-thioureido-p-benzochinondiimid eintritt, wobei erfindungsgemäß die Mengen der beiden Wirkstoffe zwischen jeweils 2 und 100 ppm im Futter betragen in Abhängigkeit von der jeweiligen Tierart.

Erfindungsgemäß können die Kombinationen der beiden genannten Wirkstoffe den Tieren vorzugsweise mit dem Futter verabreicht werden, wobei das Verhältnis der beiden Wirkstoffe über die gesamte Mastzeit konstant bleibt oder aber auch in Abbängigkeit von der nährstoffmäßigen Zusammensetzung der Futterbestandteile variiert wird oder die Kombination der Wirkstoffe wird nur in einem Teil der Mast- bzw. Aufzuchtperiode appliziert. Erfidungsgemäß können die Kombinationen der beiden Wirkstoffe in Form eines Prämix angewendet werden, wobei als physiologisch unbedenkliches Trägermaterial das Grundfutter (Standardfutter) selbst oder aber auch inerte anorganische oder organische Träger wie Calciumcarbonate, hochmolekulare Kieselsäure-Verbindungen natürlicher und synthetischer Herkunft, Zellulose, Stärke oder Milch oder Milchpulver verwendet werden können.

Durch die erfindungsgemäßen Kombinationen der beiden Wirkstoffe ergeben sich bei ihrer Anwendung Vorteile gegenüber der Verwendung der Einzelwirkstoffe. So bleibt der erzielbare Mehrsuwachs an Körpergewicht im wesentlichen konstant, oder erhöht sich sogar, wenn Kombinationen der erfindungsgemäßen Zusammensetzung angewendet werden, wobei dieses Ergebnis erfindungsgemäß dadurch erzielt wird, daß der Anteil jedes der beiden Wirkstoffe in der Kombination nur einen geringen Teil der jeweiligen Dosis bei Einzelapplikation beträgt.

Weiterhin ist der Futtereinsparungs-Effekt bei Verwendung der erfindungsgemäßen Kombinationen der gleiche oder größer als bei Verwendung der Einzelwirkstoffe, wiederum erzielt durch die erfindungsgemäße Kombination in verringerten Dosen. Ein weiterer Vorteil ist darin zu sehen, daß durch die erfindungsgemäßen Dosierverringerungen in den Kombinationen die Gefahr toxischer Nebenwirkungen stark reduziert wird, welches wieder der Tiergesundheit und damit den Mast- bzw. Aufzucht-Erfolg des gesamten mit den erfindungsgemäßen Kombinationen behandelten Tierbestandes steigert.

Weiterhin wird damit der Vorteil erzielt, daß durch erfindungsgemäßen mögliche Reduzierung der Dosen eine geringere Gesamt-Wirkstoffmenge benötigt wird, die wirtschaftlichen Aufwendungen für die Produktion der Wirkstoffe werden dadurch verringert, bei Beibehaltung oder Verbesserung des ergotropen Effekts.

**Ausführungsbeispiel**

**Beispiel 1**

Je 24 - 26 männliche Küken der Rottenauer Hybridzucht wurden in einem Gruppenfütterungsversuch in Käfighaltung über 51 Tage mit einer Grundfuttermischung mit einem Rohnährstoffgehalt von

90,06 % Trockensubstanz
5,94 % Rohasche
21,11 % Rohprotein
4,22 % Rohfaser

und mit unterschiedlichen Kombinationen von 6-Methyluracil (nachfolgend P abgekürzt) und N-Guanidino-N'-thioureido-p-benzochinon-diimid (nachfolgend A abgekürzt) gefüttert. Futter und Wasser standen den Tieren über Futterautomaten und Nippel-bzw. Durchlauftränken zur freien Verfügung. Das Versuchsergebnis wurde bestimmt durch folgende Parameter:

- durchschnittliche Lebendmasse in jeder Gruppe (in kg und als prozentuale Zunahme)
- Futteraufwand (kg Futter/kg Zunahme an Körpermasse und als prozentuele Verringerung)

Das Ergebnis zeigt Tabelle 1.

**Tabelle 1:**

| Konzentration von P und A im Futter (ppm) | Durchschnittl. Lebendmasse | % | Futteraufwand (kg/kg Zunahme) | % |
|---|---|---|---|---|
| Kontrolle | 1786 | 100 | 2,41 | 100 |
| 25 + 25 | 1910 | 107 | 2,47 | 98 |
| 50 + 20 | 1855 | 104 | 2,47 | 102 |
| 50 + 10 | 1880 | 105 | 2,36 | 98 |

**Beispiel 2**

Je 24 - 26 weibliche Briolerküken der Rottenauer Hybridzucht wurden in einem Gruppenfütterungsversuch in Bodenhaltung über 51 Tage mit einer Grundfuttermischung wie in Beispiel 1 angegeben und mit unterschiedlichen Kombinationen von P und A unter den gleichen Bedingungen wie in Beispiel 1 angegeben gefüttert. Die Auswertung erfolgte entsprechend Beispiel 1.

Tabelle 2:

| Konzentration von P und A im Futter (ppm) | Durchschnittl. Lebendmasse | % | Futteraufwand (kg/kg Zunahme) | % |
|---|---|---|---|---|
| Kontrolle | 1390 | 100 | 2,28 | 100 |
| 25 + 25 | 1450 | 104 | 2,23 | 98 |
| 50 + 20 | 1383 | 99,5 | 2,25 | 99 |
| 50 + 10 | 1437 | 103 | 2,34 | 103 |

**Beispiel 3**

In einem Vergleichsversuch wurden je 12 männliche Broilerküken der gleichen Zucht in einem Gruppenfütterungsversuch in Käfighaltung über 44 bzw. 51 Tage mit einer Grundfuttermischung wie in Beispiel 1 angegeben und mit unterschiedlichen Mengen P oder A unter den gleichen Bedingungen wie in Beispiel 1 gefüttert.Die Auswertung erfolgte entsprechend Beispiel 1. Das Ergebnis zeigt Tabelle 3.

Tabelle 3:

| Konzentration von P oder A | Durchschnittl. Lebendmasse (g) | % | Futteraufwand (kg/kg Zunahme) | % |
|---|---|---|---|---|
| Kontrolle | 1325 [1] | 100 | 2,06 [1] | 100 |
| 25 A | 1319 [1] | 99,5 | 2,05 [1] | 99,5 |
| 50 A | 1435 [1] | 108 | 1,95 [1] | 95 |
| Kontrolle | 1700 [2] | 100 | 2,36 [2] | 100 |
| 50 P | 1625 [2] | 99,1 | 2,40 [2] | 102 |
| 75 P | 1739 [2] | 102,3 | 2,34 [2] | 99 |
| 100 P | 1743 [2] | 102,5 | 2,29 [2] | 97 |
| 150 P | 1796 [2] | 105,6 | 2,19 [2] | 93 |

[1] 44 Tage Fütterungsdauer
[2] 51 Tage Fütterungsdauer

**Beispiel 4**

In einem Gruppenfütterungsversuch wurden 12 Kälbern (7 männliche und 5 weibliche) eine Mischung von 56 mg 6-Methyluracil (im folgenden mit P abgekürzt) und 28 mg N-Guanidino-N'-Thioureido-p-benzochinondiimid (im folgenden mit A abgekürzt) je Tier und Tag appliziert. Die Wirkstoffmischung wurde den Tieren als Bestandteil der Magermilchtränke gegeben. Parallel dazu wurden 3 Kontrollgruppen fast gleicher Größe und gleicher geschlechtlicher Zusammensetzung gefürt. Die Tiere einer Gruppe erhielten 48 mg Nitrovin pro Tier und Tag, der anderen Gruppe 60 mg Zinkbacitracin, während in der letzten Gruppe kein Ergotropikum appliziert wurde. Die durchschnittliche Lebendmasse aller Tiere lag zu Versuchsbeginn zwischen 39,85 kg und 40,95 kg. Die tägliche Gesamtttränkmenge wurde gleichmäßig auf zwei Mahlzeiten verteilt verabreicht. Kraftfutter und Trockengrüngut wurde den Tieren aller Gruppen zur beliebigen Aufnahme angeboten. Wägungen der Tiere erfolgten zu Beginn und am Ende des Versuchsabschnittes an jeweils zwei aufeinanderfolgenden Tagen sowie während des Versuches in Abständen von 14 Tagen.

Für die Versuchsauswertung wurden herangezogen:
- Entwicklung der Lebendmasse (LM) und die tägliche Lebendmasse-Zunahme (LMZ)
- Futteraufwand gemessen als EFr und verdaulichem Rohprotein (vRp)

Das Versuchsergebnis zeigen die Tabellen 4 und 5.

**Tabelle 4:**

Lebendmasseentwicklung (LM) und tägliche Lebendmasse-Zunahme (LMZ)

| | Gruppe I | | | Gruppe II | | |
|---|---|---|---|---|---|---|
| | LM | LMZ [1) | | LM | LMZ [1) | |
| | kg | g | % | kg | g | % |
| Versuchs-beginn | 40,40 | | | 40,95 | | |
| 2. Woche | 44,80 | 312 | 100 | 48,20 | 518 | 166 |
| 4. Woche | 52,60 | 562 | 100 | 57,70 | 677 | 120 |
| 6. Woche | 63,90 | 808 | 100 | 69,60 | 848 | 105 |
| 8. Woche | 75,20 | 804 | 100 | 81,80 | 875 | 109 |
| 10. Woche | 84,90 | 696 | 100 | 92,50 | 762 | 109 |
| 1.—10. Woche | | 634 | 100 | | 736 | 116 |

| | Gruppe III | | | Gruppe IV | | |
|---|---|---|---|---|---|---|
| | LM | LMZ [1) | | LM | LMZ [1) | |
| | kg | g | % | kg | g | % |
| Versuchs-beginn | 39,85 | | | 40,00 | | |
| 2. Woche | 46,70 | 490 | 157 | 47,70 | 551 | 177 |
| 4. Woche | 57,40 | 764 | 136 | 59,10 | 810 | 144 |
| 6. Woche | 70,60 | 939 | 116 | 73,10 | 999 | 124 |
| 8. Woche | 82,80 | 875 | 109 | 84,40 | 811 | 101 |
| 10. Woche | 93,30 | 750 | 108 | 95,40 | 787 | 113 |

**Tabelle 5:**

Aufwand an EFr und verdaulichem Rohprotein (vRp)

| | I | II (Zn-Bac.) | III (Nitrovin) | IV (A/P) |
|---|---|---|---|---|
| Efr-Aufwand | 1545 (100) | 1418 (92) | 1430 (93) | 1440 (93) |
| vRp-Aufwand | 460 (100) | 418 (91) | 420 (91) | 421 (92) |

**Beispiel 5**

In einem analogen Versuch wurden 11 Kälbern die gleiche Mange an Kombinations-Präparat A + P wie in Beispiel 1 angegeben in gleicher Applikationsart gegeben. Die Größ ße der Kontrollgruppen, ihre geschlechtliche Zusammensetzung sowie Art und Menge der anderen Ergotropika entsprachen ebenfalls dem Beispiel 1. Das durchschnittliche Gewicht der Tiere variierte in den einzelnen Gruppen zwischen 44,50 kg und 45,05 kg und lag damit höher als im Beispiel 1. Die Auswertung erfolgte wie im Beispiel 1 angegeben. Das Versuchsergebnis zeigen die Tabellen 6 und 7.

**Tabelle 6:**

Lebendmasseentwicklung (LM) und tägliche Lebendmasse-Zunahme (LMZ)

| | Gruppe I | | | Gruppe II | | |
|---|---|---|---|---|---|---|
| | LM | LMZ[1] | | LM | LMZ[1] | |
| | kg | g | % | kg | g | % |
| Versuchs-beginn | 44,5 | | | 45,0 | | |
| 2. Woche | 49,6 | 361 | 100 | 52,0 | 500 | 138 |
| 4. Woche | 59,5 | 713 | 100 | 61,8 | 695 | 97 |
| 6. Woche | 71,2 | 827 | 100 | 72,8 | 786 | 95 |
| 8. Woche | 84,1 | 927 | 100 | 85,5 | 906 | 98 |
| 10. Woche | 96,0 | 848 | 100 | 98,1 | 901 | 106 |
| 1.—10. Woche | | 735 | 100 | | 758 | 103 |

| | Gruppe III | | | Gruppe IV | | |
|---|---|---|---|---|---|---|
| | LM | LMZ[1] | | LM | LMZ[1] | |
| | kg | g | % | kg | g | % |
| Versuchs-beginn | 44,7 | | | 44,9 | | |
| 2. Woche | 51,4 | 480 | 133 | 52,6 | 551 | 153 |
| 4. Woche | 63,6 | 870 | 122 | 64,1 | 822 | 115 |
| 6. Woche | 75,3 | 832 | 101 | 77,7 | 968 | 117 |
| 8. Woche | 88,5 | 945 | 102 | 89,3 | 829 | 89 |
| 10. Woche | 102,0 | 964 | 114 | 101,2 | 851 | 100 |
| 1.—10. Woche | | 818 | 111 | | 804 | 109 |

[1] im Versuchsabschnitt

Gruppe I    ohne Zusatz
Gruppe II    + Zinkbacitracin
Gruppe III    + Nitrovin
Gruppe IV    + Kombinationspräparat A + P

**Tabelle 7:**

Aufwand an EFr und verdaulichem Rohprotein (vRp)

| | I | II (Zn-Bac.) | III (Nitrovin) | IV (A/P) |
|---|---|---|---|---|
| EFr-Aufwand | 1539 (100) | 1496 (97) | 1454 (94) | 1518 (99) |
| vRp-Aufwand | 451 (100) | 437 (97) | 423 (94) | 441 (98) |

**Beispiel 6**

60 Schweine (Masthybriden E x L x Linie F 150), die bei der Einstallung durchschnittlich je 28 kg wogen, wurden in einem Einzelfütterungsversuch über 134 Tage mit Schweinemastalleinfutter, welches mit 6-Methyluracil (im folgenden P genannt) oder N-Guanidino-N'-thioureido-p-benzochinondiimid (im folgenden A genannt) und mit einer Kombination dieser beiden Wirkstoffe versetzt war, gefüttert. Die wertbestimmenden Inhaltsstoffe des Futters Waren:

Energiegehalt (EFs/kg OS): 598 - 610
- Lysin (g/kg OS): 7,7 - 6,3
- Rohprotein (g/kg OS): 156 - 147
Die Tiere wurden vor Versuchsbeginn unter berüksichtigung ihrer Lebendmasse gleichmäßig in 5 Gruppen zu je 12 Tieren eingeteilt, darunter 3 Versuchsgruppen und 2 Kontrollgruppen. Die Tiere einer Kontrollgruppe erhielten Schweinemastalleinfutter ohne Antibiotikazusatz, die Tiere der zweiten Kontrollgruppe erhielten zu diesem Futter eine Wirkstoffmischung mit 3 % Antibiotikum-Konzentrat mit 5 % Aktivität. Die Tiere der drei Versuchsgruppen erhielten zusätzlich zum Schweinemastalleinfutter P oder A allein oder eine Kombination von P und A. Das Futter wurde trocken ad libitum verabreicht, Wasser stand den Tieren über Selbsttränken zur Verfügung. Ab 120. Versuchstag wurde die Verabreichung der Wirkstoffe P und A bzw. der Kombination von P und A eingestellt, ab diesem Zeitpunkt erhielten die Tiere dieser drei Versuchsgruppen das gleiche Futter wie die tiere der Kontrollgruppe, die von Versuchsbeginn an nur Schweinemastalleinfutter ohne Antibiotikum-Zusatz erhielten.

Das Versuchsergebnis wurde bestimmt durch folgende Parameter:
- durchschnittliche Lebendmassezunahme (g/Tier und Tag)
- Energiaufwand (KEF$_s$/kg Zunahme an Körpermasse)

Das Ergebnis zeigt Tabelle 8.

**Tabelle 8:**

| Wirkstoffe | Durchschnittl. Lebendmasse am Versuchs-ende (kg) | Durchschnittl. Lebendmasse-zunahme (g/Tier u. Tag) | rel. (%) | Energie-aufwand KEF$_s$/kg Zunahme | rel. (%) |
|---|---|---|---|---|---|
| 100 ppm A | 122,7 | 690 | 95 | 2,69 | 104 |
| 75 ppm A | 133,1 | 772 | 106 | 2,48 | 95 |
| 50 ppm P + 37,5 ppm A | 135,1 | 786 | 108 | 2,52 | 97 |
| Kontrolle (ohne Anti-biotikum) | 126,1 | 728 | *100* | 2,60 | *100* |
| Kontrolle (mit Anti-biotikum) | 130,4 | 735 | 101 | 2,58 | 99 |

## Patentansprüche

1. Verfahren zur Anwendung ergotroper Wirkstoffkombinationen in der Aufzucht und Mast von Geflügel, Schweinen und Rindern, dadurch <u>gekennzeichnet</u>, dass als Wirkstoffe 6-Methyluracil und N-Guanidino-N'-thioureido-p-benzochinondiimid verwendet werden, wobei die Kombinationen der beiden genannten Wirkstoffe jeden einzelnen Wirkstoff in Mengen von 2 bis 100 ppm im Futter enthält, und die Gesamtmenge beider Wirkstoffe 125 ppm nicht überschreitet.

2. Verfahren gemäss Anspruch 1, dadurch <u>gekennzeichnet</u>, dass die Gesamtmenge beider Wirkstoffe 100 ppm nicht überschreitet.

3. Verfahren gemäss Anspruch 1 oder 2, dadurch <u>gekennzeichnet</u>, dass die Kombinationen der beiden Wirkstoffe in Form eines Prämix auf inerten organischen oder anorganischen Trägern sowie mit oder ohne zusätzliche Stoffe, wie Vitamine und Spurenelemente, und für die Aufzucht und Mast erforderlichen Stoffe verwendet werden.

4. Verfahren nach Ansprüchen 1 bis 3, dadurch <u>gekennzeichnet</u>, dass die Gesamtmenge der beiden Wirkstoffe und ihr Mengenverhältnis in den Kombinationen wahlweise über die gesamte Aufzucht und Mastperiode konstant gehalten oder in Abhängigkeit vom Alter bzw. Gewicht der Tiere variiert wird, und dass ferner die Gesamtmenge der Wirkstoffe und ihr Mengenverhältnis in Abhängigkeit vom Energieinhalt des verwendeten Futters angewendet wird.

## Claims

1. Process for the use of ergotropic agent combinations in the breeding and the fattening of poultry, pigs and cattle, characterized in that 6-methyluracil and N-guanidino-N'-thioureido-p-benzoquinone-diimide are used as agents, the combinations of both the said agents containing each individual agent in amounts of 2 to 100 ppm in the feed, the total quantity of both agents not exceeding 125 ppm.

2. Process according to claim 1 characterized in that the total amount of both agents does not exceed 100 ppm.

3. Process according to claim 1 or 2 characterized in that the combinations of both agents are used in the form of a premix on inert organic or inorganic carriers with or without additional substances such as vitamins, trace elements and substances necessary for breeding and fattening.

4. Process according to claims 1 to 3 characterized in that the total quantity of both agents and the ratio of their quantities in the combinations may either be kept constant or varied according to the age or weight of the animals throughout the breeding and fattening period and that further the total quantity of the agents and the ratio of their quantities are dependent on the energy content of the feed used.

**0 125 322**

### Revendications

1. Procédé pour l'application des combinaisons d'agents ergotropiques dans l'élevage et l'engraissement de volailles, de porcs et de bovins, caractérisé en ce qu'on utilise, comme agents actifs, le 6-méthyluracile et le N-guanidino-N'-thiouréido-p-benzoquinone-diimide, les combinaisons des deux agents cités contenant chaque agent actif individuel en quantité de 2 à 100 ppm dans la nourriture, et la quantité totale des deux agents actifs ne dépassant pas 125 ppm.

2. Procédé selon la revendication 1, caractérisé en ce que la quantité totale des deux agents actifs ne dépasse pas 100 ppm.

3. Procédé selon la revendication 1 ou 2, caractérisé en ce que les combinaisons des deux agents actifs est utilisée sous forme d'un mélange préalable de supports inertes organiques ou inorganiques avec ou sans substances additionnelles telles que des vitamines ou des oligo-éléments, et des substances nécessaires à l'élevage et à l'engraissement.

4. Procédé selon les revendications 1 à 3, caractérisé en ce que la quantité totale des deux agents actifs et leur rapport quantitatif dans les combinaisons sont, à volonté, tenus constants pendant toute la période d'élevage et d'engraissement, ou bien sont modifiés en fonction de l'âge ou du poids des animaux, et en ce qu'on emploie en outre la quantité totale des agents actifs et leur rapport quantitatif en fonction du contenu énergétique de la nourriture utilisée.